# EUROPEAN PATENT SPECIFICATION

(11) **EP 0 916 347 B1**
(45) Date of publication and mention of the grant of the patent: **19.02.2003**
(21) Application number: 97600009.1
(22) Date of filing: 18.11.1997
(51) Int. Cl.: A61K 47/10, A61K 47/22, A61K 31/165

(54) **Pharmaceutical injectable solutions containing paracetamol and combinations of paracetamol with other active substances**
Pharmazeutische injizierbare Lösungen, die Paracetamol und Kombinationen aus Paracetamol mit anderen Aktivsubstanzen enthalten
Solutions pharmaceutiques injectables contenant du paracétamol et des associations du paracétamol avec d'autres substances actives

(43) Date of publication of application: 19.05.1999
(60) Divisional application: 02023125.4
(73) Proprietor: Uni-Pharma Kleon Tsetis A.B.E.E., Farmakeftika Ergastiria, 145 64 Kifissia (GR)
(72) Inventor: Tseti, Ioulia K., 14564 Kifissia (GR)

(56) References cited:
- WO-A-95/05812
- DE-A- 3 111 591
- GR-B- 1 001 523
- GR-B- 1 002 731
- US-A- 5 296 241

## Description

This invention refers to the pharmacotechnical field and has to do with pharmaceutical solutions of Hydroxy-acetanilide (Paracetamol) or/and its combination with other active substances suitable from the pharmacotechnical and therapeutical point of view, for parenteral use.

Paracetamol is considered to be the main active metabolite of phenacetin and acetanidile having analgesic and antipyretic properties. Paracetamol has equivalent analgesic and antipyretic action to that of aspirin whilst it expresses weak anti-inflammatory action therefore its use in inflammatory rheumatic diseases is limited.

The mechanism of its analgesic action is still unclarified. It is believed that it mainly acts by inhibiting prostaglandins biosynthesis and to a lesser extent by peripherically inhibiting algogenic stimulus origin. The peripheral action is due also to inhibition of proglandins biosynthesis or to inhibition or to other endogenous substances action that sensitize pain's receptors after mechanic or chemical stimulation.

As far as its antipyretic action is concerned, Paracetamol induces temperature fall to feverish but not to normal subjects.
It is believed that the antipyretic effect of Paracetamol is due to central action on the temperature controlled centre of hypothalamus resulting in peripheral vasodilation leading to skin peripheral blood flow increase, perspiration and temperature loss.

This peripheral action of Paracetamol is due also to prostaglandins biosynthesis inhibition into hypothalamus.

Paracetamol administered in recommended dosage does not exert any effect of the cardiovascular and respiratory system nor provokes acid-base balance disorders.
Several studies have confirmed the effectiveness and safety of Paracetamol's parenteral administration.
Paracetamol is well absorbed when intramuscularly administered and its blood level is similar to that obtained after its oral administration.

The absorption rate is slower of that obtained when Paracetamol is orally administered, resulting in desirable blood levels for more prolonged time.

There is also another advantage of injectable Paracetamol since the 20% loss of the drug that is observed after oral administration doesn't exist (Macheras et al. 1989, Pharmaceutical Codex 1994).

Paracetamol is metabolized by the microsomal enzymes of the liver and 95% of it is excreted through urines as conjugated derivatives of sulfuric (35%) and glucouronic acids (60%) whilst only 2% is excreted unchangeable (Gillette 1981, Clissold 1986, Remington 1990, Insel 1992, AMA-DE 1994).

Also a small part of Paracetamol, approx. 3%, is oxidized by the liver cytochrome P-450 to a toxic intermediate metabolite that is connected to the liver deposit of glutathione, producing finally a non-toxic combination, which is excreted conjugated with cysteine and mercapturic acid (Mitchell et al. 1982, Jackson et at. 1984, Remington 1990, Insel 1992).

Fifteen years ago, combinations of Paracetamol plus other active substances as i.e. analgesics (aspirin, codeine), spasmolytics (hyoscine-N-butyl-bromide, mebeverine etc), or substances antidote for Paracetamol's toxicity were administered orally or rectally but not parenterally (intramuscularly or intravenously).

Documents GR-B-1001523, GR-B-1002731 and WO-A-9505812 are related to injectable parental solutions including paracetamol dissolved in Ethanol, Glycerol formal and water.

Therefore, preparation of parenteral solutions of Paracetamol and combinations of it with other substances as above mentioned, are considered to be indispensable for use in the modern therapeutics for a quicker and greater therapeutical effect.

Whilst Paracetamol is soluble in many organic solvents, however solutions of Paracetamol with such solvents are unfit for therapeutical use, because of the produced toxicity when parenterally administered (intramuscularly or intravenously) and because of the present technical problems as i.e. chemical instability leading to precipitates, low fluidity etc.

As above, the preparations of injectable solutions of Paracetamol and combinations of Paracetamol with other active substances require the choice of the suitable solvent or combination of solvents, comprising also water, reciprocating to certain requirements of suitability as : to be pharmacologically inactive, to not form complexes with the active substance, to be blood conventional, free of sensitization or irritating activity, chemically stable, clear and not influenced by pH declinations.

But it is important the selected solvents to not interfere with Paracetamols' or other's substances therapeutical properties.
From the pharmacotechnical point of view, the selected solvent or solvents system must have the full ability of mixing with water not only because this way it or they will facilitate the manufacturing process but will also reduce the manufacturing cost.

Furthermore, this property of fully mixing with water makes depended to a great extend the pharmacokinetic and bio-availability of the preparation and the local tolerance in the site of injection as well.

According to the present invention chemically stable solutions of Paracetamol are obtained, suitable for parenteral administration as well as solutions of Paracetamol with other active substances as i.e. with Codeine for a preparation with increased analgesic effect or with Hyoscine-N-Butylbromide for a preparation having spasmo-analgetic effect.

Since Paracetamol is not soluble in the water, efforts made for its dissolution into organic-solvents or mixtures of them, suitable for parenteral use.

Among the tested solvents proved suitable Ethanol, Benzylic Alcohol and GLYCEROL FORMAL Ethanol and Benzylic Alcohol are already used as solvents for parenteral administration of drugs, whilst GLYCEROL FORMAL (an almost atoxic substance having LD₅₀ to rats 3,5mg/kg body weight, when is given intravenously), having the property to be mixed in whichever rate with water and/or with Ethanol is the first time that has been applied in the preparation of parenteral use solution.

Our experiments for the discovery of proper solvents for Paracetamol's solubilization, have shown that there is a system in which other active substances that may be combined with Paracetamol for therapeutical purposes are also soluble.

**Solvent mixture consisting of Benzylic Alcohol-Glycerol Formal - Water in the ratio of 10:50:40 respectively** (according to the invention as claimed) To the mixture of these three solvents into which Paracetamol is easily dissolved were added the following matters : LIDOCAINE HCl (local anaesthetic) DISODIUM EDETATE, SODIUM METABISUL-FITE (antioxidant agent) and DISODIUM DIBASIC PHOSPHATE as pH buffer which must oscillate between 5,5-5,6.

The occurred final solution after mixing and dissolution of the auxiliary matters is clear, chemically stable and atoxic satisfying all requirements for a safe parenteral administration.

| **Composition of a 4 ml ampoule** | |
|---|---|
| PARACETAMOL | 600,0 mg |
| LIDOCAINE HCI | 20,0 mg |
| DISODIUM EDETATE | 4,0 mg |
| DISODIUM DIBASIC PHOSPHATE | q.s. |
| SODIUM METABISULFITE | 2,0 mg |
| GLYCEROL FORMAL | 1,5 mg |
| BENZYL ALCOHOL | 0,4 mg |
| WATER | q.s. |

If to the mixture consisting of Benzylic Alcohol - GLYCEROL FORMAL - Water into which Paracetamol is soluble HYOSCINE-N-BUTYLBROMIDE and the auxiliary matters LIDOCAINE HCl, DISODIUM EDETATE, DISODIUM DIBASIC PHOSPHATE, NIPAGIN A and NIPASOL M, are added, then we obtain a clear, chemically stable atoxic solution, suitable for parenteral administration, combining the analgetic action of Paracetamol and the spasmolytic effect of HYOSCINE-N-BUTYLBROMIDE for the confrontation of painful spastic conditions of splachnic organs.

| **Composition of a 4 ml amp. solution** | | |
|---|---|---|
| PARACETAMOL | 15% | 600,0 mg |
| HYOSCINE-N-BUTYLBROMIDE | 0,5% | 20,0 mg |
| LIDOCAINE HCl | 0,5% | 20,0 mg |
| DISODIUM EDETATE | 0,01% | 0,4 mg |
| DISODIUM DIBASIC PHOSPHATE | q.s.% | q.s. |
| NIPAGIN A | 0,13% | 1,8 mg |
| NIPASOL M | 0,08% | 0,2 mg |
| BENZYL ALCOHOL | 10% | 0,4 ml |
| GLYCEROL FORMAL | 37,5% | 1,5 ml |
| WATER q.s. ad. | 100ml | q.s. |

The combination of Paracetamol and Codeine in tablet or elixir for the treatment of painful conditions that do not respond to mild analgesics, is pharmacologically accepted but up to now there is not available injectable preparation of the above mentioned combination

To the "basic" solution of injectable Paracetamol as it has been above described, Codeine PHOSPHATE was added and clear, chemically stable solutions were obtained suitable for parenteral administration comprising Paracetamol 600 mg and 30 or 15 mg Codeine respectively in every 4 ml amp.
1. Solution suitable for parenteral administration of matters not soluble in water consisting of 1) ETHANOL, GLYCEROL FORMAL and Water and 2) BENZYLIC ALCOHOL-GLYCEROL FORMAL and Water.
2. Method for the preparation of injectable solution of Paracetamol into aqueous organic solution where solvents of the solution are ETHANOL, GLYCEROL FORMAL and Water, in the ratio 10:50:40 by volume.
3. The solution of item 2 comprises as excipients LIDOCAINE HCI (local anaesthetic) 5mg/ml, DISODIUM EDETATE 0,10mg/ml, DISODIUM DIBASIC PHOSPHATE (pH buffer) q.s. for pH 5,5-5,6, SODIUM METABISULFITE 0,5mg/ml.
4. Process for the preparation of injectable solution of Paracetamol into aqueous organic solution, where solvents consisting the solution are BENZYLIC ALCOHOL, GLYCEROL FORMAL and Water, in a ratio of 10:50:40 by volume.
5. Process for the preparation of injectable solution of Paracetamol and HYOSCINE-N-BUTYLBROMIDE based on the solution of item 2 and the excipients of item 3, after the elimination of SODIUM METABISULFITE and addition of NIPAGIN A and NIPASOL M.
1. Process for preparation of injectable solution of Paracetamol and HYOSCINE-N-BUTYLBROMIDE based on the solution of item 4 and the excipients of item 5.
7. Process for the preparation of injectable combination solution of Paracetamol plus CODEINE PHOSPHATE according to items 1, 3 and 5.

## Claims

1. Solution suitable for parenteral administration, comprising paracetamol and benzyl alcohol, glycerol formal and water.

2. Solution according to claim 1 wherein the ratio of benzyl alcohol:glycerol formal:water is 10:50:40 by volume.

3. Solution according to claim 1 comprising hyoscine-N-butylbromide, lidocaine HCl, disodium edetate, disodium dibasic phosphate, nipagin A and nipasol M.

4. Solution according to anyone or any combination of claims 1-3, including active substances such as hyoscine-N-butylbromide or codeine phosphate.

5. Solution according to claim 1 suitable for 4ml ampoule, comprising 600mg paracetamol, 20mg lidocaine HCl, 4mg disodium edetate, disodium dibasic phosphate q.s., 2mg sodium metabisulfite, 1.5mg glycerol formal, 0.4mg benzyl alcohol, q.s. water, said solution optionally, comprising both hyoscine-N-butylbromide as well as the mixture nipagin A and nipasol M.

6. Solution according to claim 1 suitable for 4ml ampoule solution comprising 600mg paracetamol, 20mg hyoscine-N-butylbromide, 20mg lidocaine HCl, 0.4mg disodium edetate, disodium dibasic phosphate q.s., 1.8mg nipagin A, 0.2mg nipasol M, 0.4ml benzyl alcohol, 1.5ml glycerol formal, water q.s. said solution optionally comprising 30mg or 15mg codeine phosphate.

## Patentansprüche

1. Lösung, geeignet für parenterale Verwendung, die Parazetamol und Benzylalkohol, Formalglyzerol und Wasser enthält.

2. Lösung gemäß Anspruch 1, in der das Volumenverhältnis Benzylalkohol:Formalglyzerol:Wasser 10:50:40 beträgt.

3. Lösung gemäß Anspruch 1, die Hyoscine-N-Butylbromid, Lidocain HCI, Disodiumedetat, Disodium Dibasic Phosphat, Nipagin A und Nipasol M enthält.

4. Lösung gemäß einem oder mehreren der Ansprüche 1-3, die aktive Substanzen wie Hyoscine-N-Butylbromid oder Phosphatkodein enthält.

5. Lösung gemäß Anspruch 1, geeignet für 4mL-Ampullen, die 600mg Parazetamol, 20mg Lidocain HCl, 4mg Disodiumedetat, Disodium Dibasic Phosphat q.s., 2mg Sodium Metabisulfit, 1.5mg Formalglycerol, 0.4mg Benzylalkohol, q.s. Wasser enthält, die o.g. Lösung kann sowohl Hyoscine-N-Butylbromid als auch eine Mischung aus Nipagin A und Nipasol M enthalten.

6. Lösung gemäß Anspruch 1, geeignet für Lösung für 4mL-Ampullen, die Lösung kann 600mg Parazetamol, 20mg Hyoscine-N-Butylbromid, 20mg Lidocain HCI, 0.4mg Disodiumedetat, Disodium Dibasic Phosphat q.s., 1.8mg Nipagin A, 0.2mg Nipasol M, 0.4mg Benzylalkohol, 1.5ml Formalglyzerol, Wasser enthalten, die o.g. Lösung kann 30mg oder 15mg Kodeinphosphat enthalten.

## Revendications

1. Solution convenable pour administration parentérale comprenant du paracétamole et de l'alcool benzylique, du glycérol formale et de l'eau.

2. Solution selon la revendication 1, dans laquelle le rapport alcool benzylique:glycerole formale:eau est 10:50:40 en volume.

3. Solution selon la revendication 1, comprenant de l'hyoscine-N-butylbromide, du lidocaine HCl, du disodium edetate, du disodium dibasique phosphate, du Nipagin A et du Nipasol M.

4. Solution selon n'importe quelle revendication ou n'importe quelle combinasion de revendications 1-3 comprenant des substances actives comme de l'hyoscine-N-butylbromide ou codeine phosphate.

5. Solution selon la revendication 1, convenable pour des ampoules à 4ml comprenant 600mg de paracétamole, 20mg de lidocaine HCI, 4mg disodium edetate, du disodium dibasique phosphate q.s., 2mg de sodium métabisulfite, 1.5mg de glycerole formale, 0.4mg de l'alcool benzylique, q.s. de l'eau, la dite solution comprenant de manière facultative à la fois de l'hyoscine-N-butylbromide ainsi de le mélange Nipagin A et Nipasol M.

6. Solution selon la revendication 1, convenable pour une solution dans une ampoule à 4ml comprenant 600mg de paracétamole, 20mg de l'hyoscine-N-butylbromide, 20mg de lidocaine HCI, 0.4mg de disodium edetate, du disodium dibasique phosphate q.s., 1.8mg de Nipagin A, 0.2mg Nipasol M, 0.4mg de l'alcool benzylique, 1.5ml de glycérole formale, de l'eau q.s., la dite solution contenant de manière facultative 30mg ou 15mg de codéine phosphate.
